# EUROPEAN PATENT APPLICATION

(11) **EP 4 538 359 A1**
(43) Date of publication of application: **16.04.2025**
(21) Application number: 24204746.2
(22) Date of filing: 04.10.2024
(51) Int. Cl.: C12M 1/00, C12M 3/00, C12M 1/36

(54) **CELL TREATMENT APPARATUS AND CELL TREATMENT METHOD**

(30) Priority: 06.10.2023 JP 2023174697
(71) Applicant: CANON KABUSHIKI KAISHA, Tokyo 146-8501 (JP); Canon Medical Systems Corporation, Otawara-shi, Tochigi 324-8550 (JP)
(72) Inventor: KAWANO, Takaaki, Tokyo, 146-8501 (JP); UCHINO, Shota, Tokyo, 146-8501 (JP); SAKAI, Tetsuya, Tokyo, 146-8501 (JP)
(74) Representative: Marks & Clerk LLP

(57) **Abstract**

According to one embodiment, a cell treatment apparatus (3) includes a conveyance mechanism (12), a liquid feed control unit (11, 101) and a connection control unit (103). The conveyance mechanism (12) is capable of moving a cartridge (20) including a plurality of connection ports (201). The liquid feed control unit (11, 101) is connected to at least one of the plurality of connection ports (201) and is configured to control feed of a liquid in the cartridge (20). The connection control unit (103) is configured to move at least one of the liquid feed control unit (11, 101) and the cartridge (20) when connecting the liquid feed control unit (11, 101) and the cartridge (20).

## Description

### FIELD

Embodiments described herein relate generally to a cell treatment apparatus and a cell treatment method.

### BACKGROUND

Along with advances in regenerative medicine, there are needs to automate cell treatment and a culture process to inexpensively manufacture high-quality cells. If automating cell treatment and a culture process, a flow channel is assumed to be formed using a pipe such that a culture medium can automatically be exchanged. In this case, considering a risk of liquid leakage during liquid feed caused by the pipe detached by an unexpected pressure, a safety cabinet or an additional facility corresponding to BSL-2 is preferably prepared, but it is expensive.

As a method without the necessity of installing an additional facility as described above, a process is automated using a disposal cartridge with a pipe having a double structure. When automating a process using a cartridge, if units configured to drive liquid feed are provided on the apparatus side as many as the number of liquid feed steps, the cost increases, and it is impossible to manufacture cells at low cost.

### SUMMARY

In relation to foregoing embodiments, the following matters are disclosed as one aspect and selected features of the present invention.

In an embodiment, a cell treatment apparatus includes a conveyance mechanism capable of moving a cartridge including a plurality of connection ports; a liquid feed control unit connected to at least one of the plurality of connection ports and configured to control feed of a liquid in the cartridge; and a connection control unit configured to move at least one of the liquid feed control unit and the cartridge when connecting the liquid feed control unit and the cartridge.

In an embodiment, the cell treatment apparatus may further comprise one or more valve opening/closing units each configured to open/close a plurality of valves arranged in the cartridge to switch a flow channel of the liquid arranged in the cartridge.

In an embodiment, the liquid feed control unit may include a liquid feeding pad that is a portion connected to one of the connection ports and is movable, and the liquid feed control unit may move at least one of the liquid feed control unit and the cartridge such that one or more target valves necessary for switching of the flow channel among the plurality of valves faces the valve opening/closing units in accordance with a connecting point where the liquid feeding pad is connected to one of the connection ports.

In an embodiment, the valve opening/closing units may be immovably arranged.

In an embodiment, a minimum interval between the connection ports may be an integer multiple of a minimum interval between the valves.

In an embodiment, a maximum moving distance of the liquid feeding pad may be shorter than a maximum array length of the plurality of connection ports arranged in the cartridge.

In an embodiment, the liquid feed control unit may execute the liquid feed by suction and pressure feed.

In an embodiment, the valve opening/closing units may be two-dimensionally arrayed in a matrix.

In an embodiment, the number of liquid feeding pads may be smaller than the number of the plurality of connection ports.

In an embodiment, there is provided a cell treatment method using a cell treatment apparatus including a conveyance mechanism capable of moving a cartridge including a plurality of connection ports, and a liquid feed control unit connected to at least one of the plurality of connection ports and configured to control feed of a liquid in the cartridge, wherein the method includes moving at least one of the liquid feed control unit and the cartridge when connecting the liquid feed control unit and the cartridge.

In an embodiment, the method may further include opening and closing a plurality of valves arranged in the cartridge by using one or more valve opening/closing units, in order to switch a flow channel of the liquid arranged in the cartridge.

In an embodiment, at least one of the liquid feed control unit and the cartridge may move such that one or more target valves necessary for switching of the flow channel among the plurality of valves faces the valve opening/closing units in accordance with a connecting point where a liquid feeding pad is connected to one of the connection ports, the liquid feeding pad being a portion connected to one of the connection ports and being movable.

In an embodiment, a cell treatment system includes a cartridge including a plurality of connection ports arranged in line along a longitudinal direction; a conveyance mechanism capable of moving the cartridge; a liquid feed control unit connected to at least one of the plurality of connection ports and configured to control feed of a liquid in the cartridge; and a connection control unit configured to move at least one of the liquid feed control unit and the cartridge when connecting the liquid feed control unit and the cartridge.

In an embodiment, the cartridge may include a plurality of valves configured to switch a flow channel of the liquid arranged in the cartridge, and if the cartridge is divided into a grid, the plurality of valves may be arranged on the flow channel at positions corresponding to intersections of the grid.

In an embodiment, the cell treatment system may further include a manifold including pipes in a number equal to the number of the plurality of connection ports, whose one ends are connected to the plurality of connection ports, respectively, and whose other ends are integrally arranged, and the liquid feed control unit may include a liquid feeding pad that is a portion connected to at least one of the other ends of the pipes and is movable.

### BRIEF DESCRIPTION OF THE DRAWINGS

FIG. 1 is a block diagram showing a cell treatment system according to an embodiment.
FIG. 2 is a view showing an example of the configuration of the cell treatment system according to an embodiment.
FIG. 3 is a view showing an example of the internal structure of a cartridge according to an embodiment.
FIG. 4 is a sectional view of the cartridge taken along a line A - A' in FIG. 3.
FIG. 5 is a plan view of the cartridge showing an example of the arrangement of connection ports.
FIG. 6 is a view showing the relationship between the interval between connection ports and the arrangement of valves.
FIG. 7 is a view showing an example of valve positions arranged in the cartridge.
FIG. 8 is a view showing an example of the arrangement of valve opening/closing mechanisms.
FIG. 9 is a view showing a first positional relationship between the valve positions and the valve opening/closing mechanisms in the cartridge at the time of valve opening/closing control.
FIG. 10 is a view showing a second positional relationship between the valve positions and the valve opening/closing mechanisms in the cartridge at the time of valve opening/closing control.
FIG. 11 is a flowchart showing liquid feed control processing of the cell treatment apparatus.
FIG. 12 is a diagram corresponding to moving processing of step SA1 in FIG. 11.
FIG. 13 is a diagram corresponding to connection processing of step SA1 in FIG. 11.
FIG. 14 is a diagram corresponding to valve opening/closing processing of step SA2 and suction processing of step SA3 in FIG. 11.
FIG. 15 is a diagram corresponding to valve opening/closing processing of step SA4 and pressure feeding processing of step SA5 in FIG. 11.
FIG. 16 is a view showing an example of a magnet valve in a case in which a valve is in a closed state.
FIG. 17 is a view showing an example of a magnet valve in a case in which a valve is in an open state.

### DETAILED DESCRIPTION

In general, according to one embodiment, a cell treatment apparatus includes a conveyance mechanism, a liquid feed control unit and a connection control unit. The conveyance mechanism is capable of moving a cartridge including a plurality of connection ports. The liquid feed control unit is connected to at least one of the plurality of connection ports and is configured to control feed of a liquid in the cartridge. The connection control unit is configured to move at least one of the liquid feed control unit and the cartridge when connecting the liquid feed control unit and the cartridge.

A cell treatment apparatus, cell treatment method and a cell treatment system according to this embodiment will now be described with reference to the accompanying drawings. In the following embodiment, the same reference numerals denote parts that perform the same operations, and a repetitive description will appropriately be omitted.

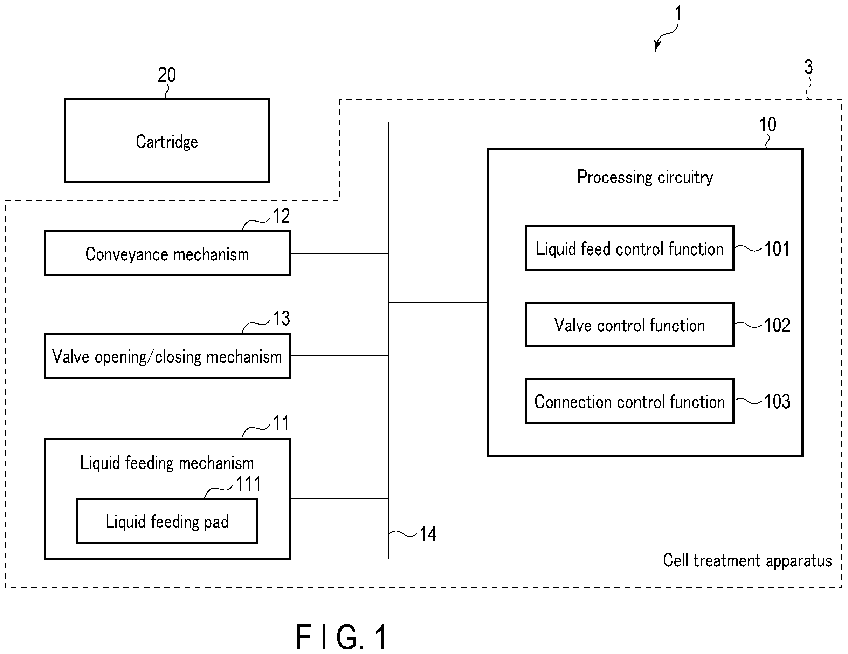

A cell treatment system according to this embodiment will be described with reference to the block diagram of FIG. 1.

A cell treatment system 1 according to this embodiment includes a cell treatment apparatus 3 and a cartridge 20. The cell treatment apparatus 3 includes a control device 10, a liquid feeding mechanism 11, a conveyance mechanism 12, and a valve opening/closing mechanism 13, and these are connected by control signal lines 14.

The control device 10 is, for example, a processing circuitry such as a processor and includes a liquid feed control function 101, a valve control function 102, and a connection control function 103. The liquid feeding mechanism 11 includes a liquid feeding pad 111. Note that the liquid feed control function 101 and the liquid feeding pad 111 are also called a liquid feed control unit together.

The liquid feed control function 101 controls, via the liquid feeding mechanism 11, feed of a liquid such as a culture solution or blood in the cartridge 20.

The valve control function 102 controls, via the valve opening/closing mechanism 13, opening/closing of a plurality of valves arranged in the cartridge 20.

When connecting the liquid feed control unit and the cartridge, the connection control function 103 controls to move at least one of the liquid feed control unit and the cartridge 20.

The liquid feeding mechanism 11 feeds the liquid in the cartridge 20 by suction and pressure feed. The liquid feeding pad 111 of the liquid feeding mechanism 11 is a portion connected to at least one of a plurality of connection ports of the cartridge 20 to be described later and is movable.

The conveyance mechanism 12 on which the cartridge 20 is loaded movably conveys the cartridge 20.

The valve opening/closing mechanism 13 opens/closes the plurality of valves arranged in the cartridge 20 in order to switch a liquid flow channel arranged in the cartridge 20.

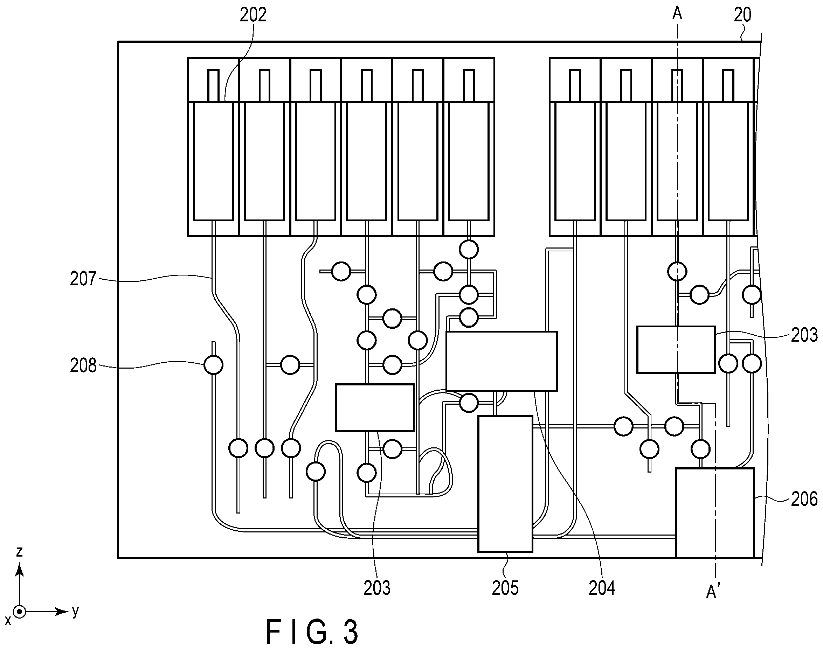

The cartridge 20 stores a culture solution, blood, a culture container, and the like in a housing, and steps from blood corpuscle separation to purification and expansion culture of CD34 positive cells, initializing factor introduction, and culture are implemented in the cartridge. Details of the cartridge 20 will be described with reference to drawings from FIG. 3.

Note that a case where cells are cultured will be described below as an example, and a culture treatment can similarly be implemented even for a biological tissue. Examples of cells include not only various cultured cell lines such as CHO cells derived from Chinese hamster ovary, mouse connective tissue L929 cells, mouse skeletal myoblast cells (C2C12 cells), normal diploid fibroblast cells (TIG-3 cells) derived from human embryonic lung, cells (HEK293 cells) derived from human embryonic kidney, A549 cells derived from adenocarcinoma of human alveolar basal epithelium, and Hela cells derived from human cervical cancer but also epithelial cells and endothelial cells forming tissues and organs in a living body, skeletal muscle cells, smooth muscle cells, and myocardial cells exhibiting contractility, neuronal cells, glial cells, and fibroblast cells forming a nervous system, liver parenchymal cells, liver nonparenchymal cells, and fat cells involved in metabolism of a living body, various kinds of stem cells such as iPS cells, embryonic stem (ES) cells, embryonic germ (EG) cells, embryonal carcinoma (EC) cells, mesenchymal stem cells, hepatic stem cells, pancreatic stem cells, skin stem cells, muscle stem cells, and germline stem cells and precursor cells of tissues, which are cells having differentiation potential, and cells differentiated and induced therefrom.

Note that the cell treatment system 1 may further include a display configured to display data concerning a cell treatment, and an input/output interface configured to output the data to the outside and obtain an input from a user, although these are not shown. The display may be included in the cell treatment apparatus 3 or may be arranged in another room if it can receive data from the cell treatment apparatus 3.

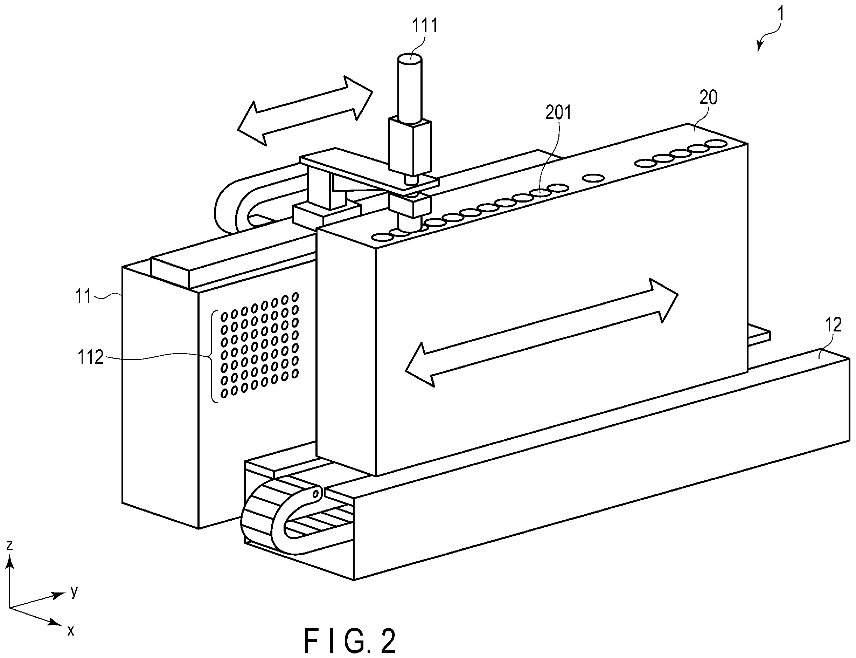

An example of the configuration of the cell treatment system 1 according to this embodiment will be described next with reference to FIG. 2.

FIG. 2 shows an example of the configuration of the cell treatment system 1 showing a state in which the cartridge 20 is mounted on the conveyance mechanism 12. The conveyance mechanism 12 is, for example, a belt conveyor and linearly guides the cartridge 20 in the y-axis direction. Note that the conveyance mechanism 12 is not limited to the belt conveyor, and may be of a guide rail type capable of sliding the cartridge 20 in the y-axis direction or a rack and pinion type. Implementing the conveyance mechanism 12 be a type capable of moving the cartridge 20 in the y-axis direction suffices.

On the upper surface of the cartridge 20, a plurality of connection ports 201 that are the connection target of the liquid feeding pad 111 are provided along the longitudinal direction. The liquid feeding pad 111 is formed to be movable in the y-axis direction, that is, the longitudinal direction of the cartridge 20. The liquid feeding pad 111 is connected to the connection port 201 of the connection target among the plurality of connection ports 201 by the connection control function 103.

Note that in the example shown in FIG. 2, a member capable of moving along a rail extending in the y-axis direction is arranged, and the liquid feeding pad 111 is connected to the member and can move in the y-axis direction. Note that the present invention is not limited to this, and an articulated driving mechanism such as a robot arm may be used if the liquid feeding pad 111 is installed in a form movable in the y-axis direction. FIG. 2 shows a case where one liquid feeding pad 111 is provided, but a plurality of liquid feeding pads 111 may be installed. In this case, the plurality of liquid feeding pads 111 may move integrally or may be independently movable.

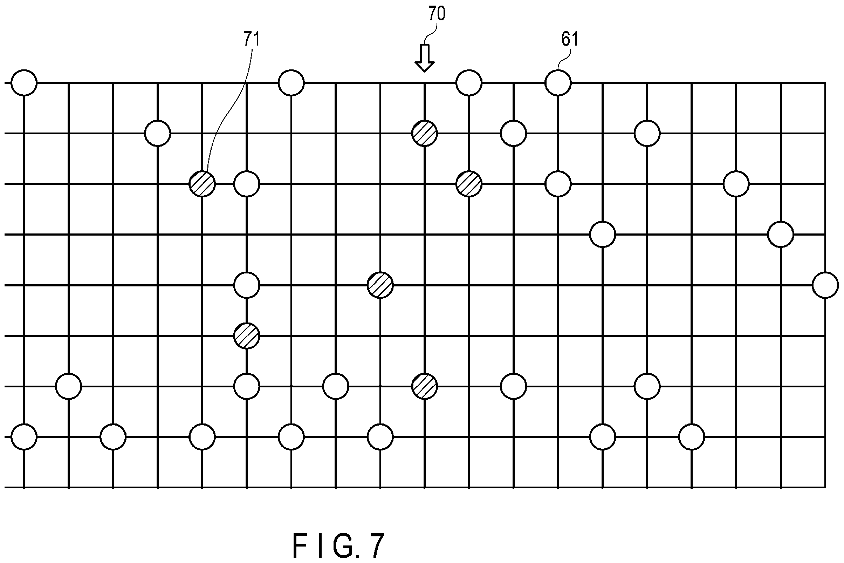

The valve opening/closing mechanism 13 is formed on a surface facing the valves arranged in the cartridge 20. The valve opening/closing mechanism (also referred to as a valve opening/closing unit) 13 is assumed to be immovably arranged. The expression "immovably arranged" means that the valve opening/closing mechanism 13 is kept stationary (i.e. does not move) with respect to the space where the cell treatment apparatus 3 is placed when at least one of the liquid feed control unit 11 and the cartridge 20 is moved. Here, a plurality of valve opening/closing mechanisms 13 are two-dimensionally arrayed in a matrix, and the valve opening/closing mechanisms 13 open/close the valves in the cartridge 20 which need to be opened/closed. Details will be described later with reference to FIGS. 7 to 9.

Note that the mounted state of the cartridge 20 is not limited to the example shown in FIG. 2. For example, defining that the example of FIG. 2 shows a state in which the cartridge 20 stands, the cartridge 20 may be mounted and moved in a laid state, that is, in a state in which the openings of the connection ports are made to face not in the z-axis direction but in the x-axis direction. In this case, control can similarly be performed if the liquid feeding pad 111 and the valve opening/closing mechanisms 13 are installed according to the direction of the cartridge.

An example of the internal structure of the cartridge 20 will be described next with reference to FIG. 3.

FIG. 3 is a perspective view showing the cartridge 20 viewed from the x-axis direction in FIG. 2. In the cartridge 20, the members are arranged from one end portion to the other end portion such that steps from blood corpuscle separation to CD34 positive cell purification, initializing factor introduction, and culture are sequentially performed in the housing of the cartridge 20. In the example shown in FIG. 3, only portions associated with the blood corpuscle separation step and the CD34 positive cell purification step will be described. More specifically, the cartridge 20 includes, in the housing, an intermediate container 202, a filter 203, a spiral flow channel 204, a recovery container 205, a culture container 206, a flow channel 207, and a valve 208. The cartridge 20 has at least a double structure formed by the flow channel 207 and the housing with respect to a liquid that flows inside. Even if the flow channel 207 breaks, the liquid is confined in the housing of the cartridge 20, and it is therefore possible to prevent a risk of infection or the like.

The intermediate container 202 is a container that temporarily holds a liquid such as a culture solution or blood in a liquid feeding step.

The filter 203 is a filter used to, for example, when separating red blood cells and white blood cells in blood corpuscle separation, leave white blood cells with a large particle size.

The spiral flow channel 204 is a flow channel having a spiral shape and is configured to separate red blood cells and white blood cells. More specifically, a suspension containing red blood cells and white blood cells is made to flow through the spiral flow channel 204, thereby centrifugally separating the red blood cells and the white blood cells.

The recovery container 205 is a container that recovers red blood cells.

The culture container 206 is a container configured to extract CD34 positive cells from the separated white blood cells and to perform expansion culture.

The flow channel 207 is made of, for example, a tubular flexible material formed by a silicone tube or a polyethylene tube, and is arranged in the cartridge 20 to feed a necessary liquid (physiological saline, suspension, or the like) and cells for each step.

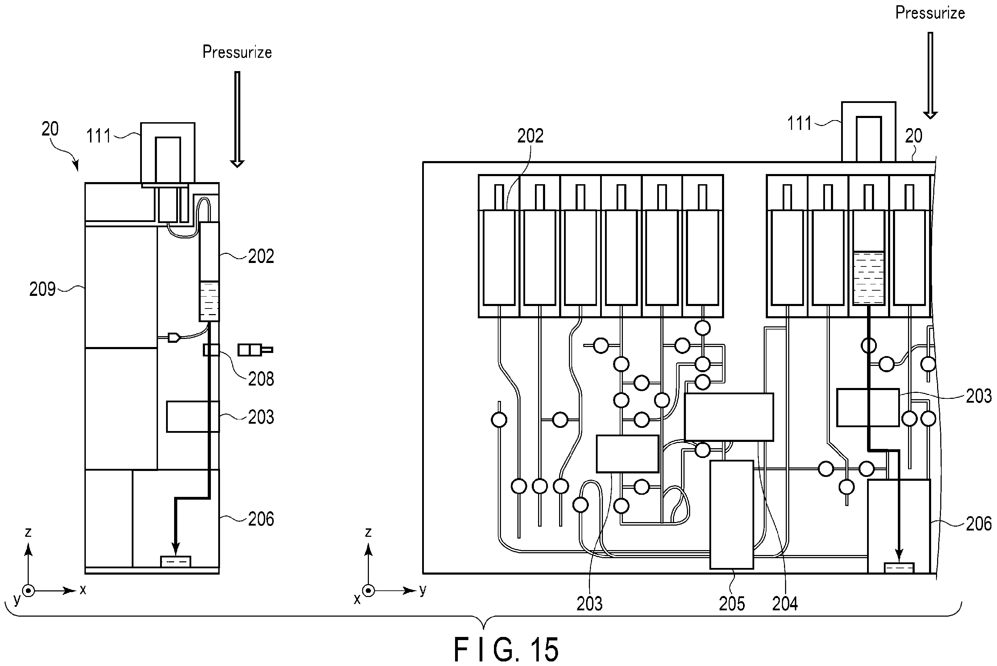

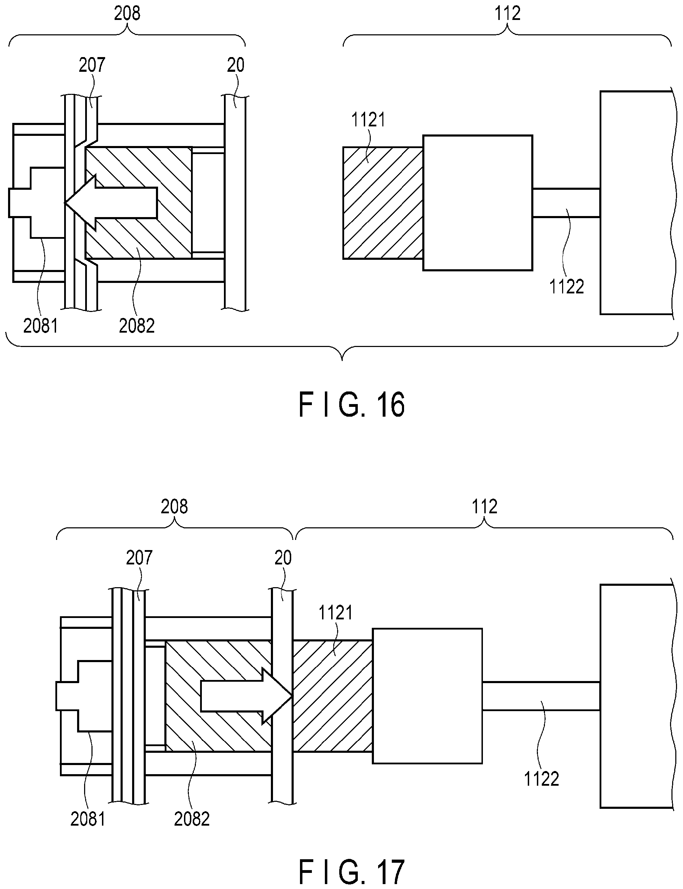

The valve 208 is "closed" to close the flow channel 207 and "opened" to open the flow channel 207. Details of the valve will be described later with reference to FIGS. 15 and 16. Note that a liquid feeding path in each step is decided by controlling opening/closing of the valve 208 in accordance with a cell treatment process and thus releasing or closing the flow channel 207.

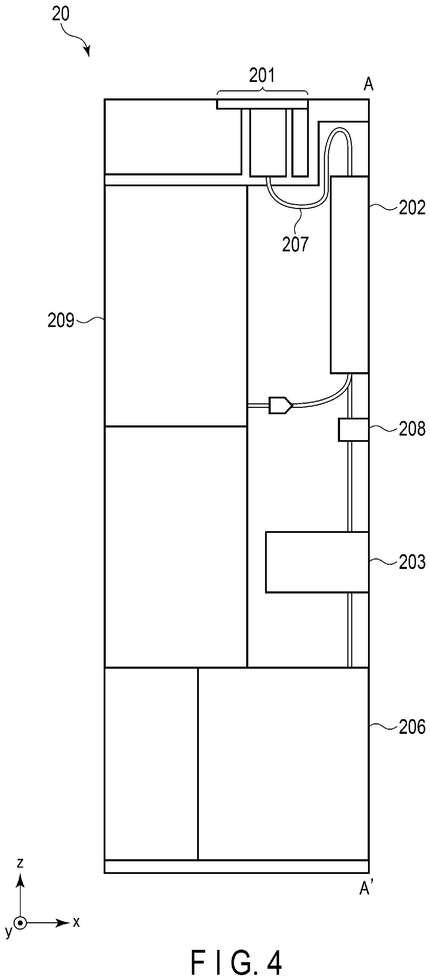

The sectional view of the cartridge 20 taken along a line A - A' in FIG. 3 will be described next with reference to FIG. 4.

FIG. 4 is a view of the cross section of the cartridge 20 taken along the line A - A', which is viewed from the y-axis direction. The cartridge 20 includes a liquid pack storage portion 209 in addition to the components shown in FIGS. 2 and 3.

The liquid pack storage portion 209 is a region in the housing of the cartridge 20, in which, for example, a blood pack, a culture solution pack, a physiological saline pack, or the like is stored.

The connection port 201 has an upper portion connected to the liquid feeding pad 111, and is formed as an air chamber in the housing of the cartridge 20. The connection port 201 includes a filter arranged on the upper portion to prevent fungi, bacteria, dust, and the like in air from mixing into the cartridge 20.

In the example shown in FIG. 4, the intermediate container 202, the spiral flow channel 204, the culture container 206, the liquid pack storage portion 209, and the connection port 201 are connected by the flow channel 207.

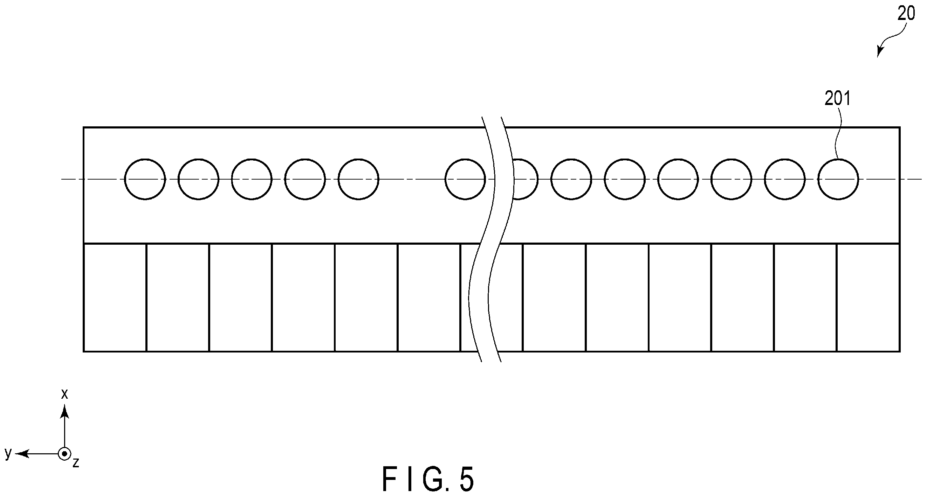

An example of the arrangement of the connection ports 201 formed on the upper surface of the cartridge 20 will be described next with reference to FIG. 5.

As shown in FIG. 5, the plurality of connection ports 201 are arranged in line along the longitudinal direction of the cartridge 20. Since the liquid feeding pad 111 need only move in one direction when connecting to each connection port 201, the driving mechanism of the liquid feeding mechanism 11 and the conveyance mechanism 12 can be simplified.

Note that the connection ports 201 need not always be arranged in line as shown in FIG. 5, and may be arranged in multiple lines of two or more lines. That is, any arrangement can be adopted if the liquid feeding pad 111 can be connected to a predetermined connection port 201 by cooperation of the liquid feeding mechanism 11 and the conveyance mechanism 12.

The maximum moving distance of the liquid feeding pad 111 in the y-axis direction is designed to be shorter than the maximum array length of the plurality of connection ports 201 arranged in the cartridge 20. This can make the driving mechanism of the liquid feeding pad 111 compact and simple.

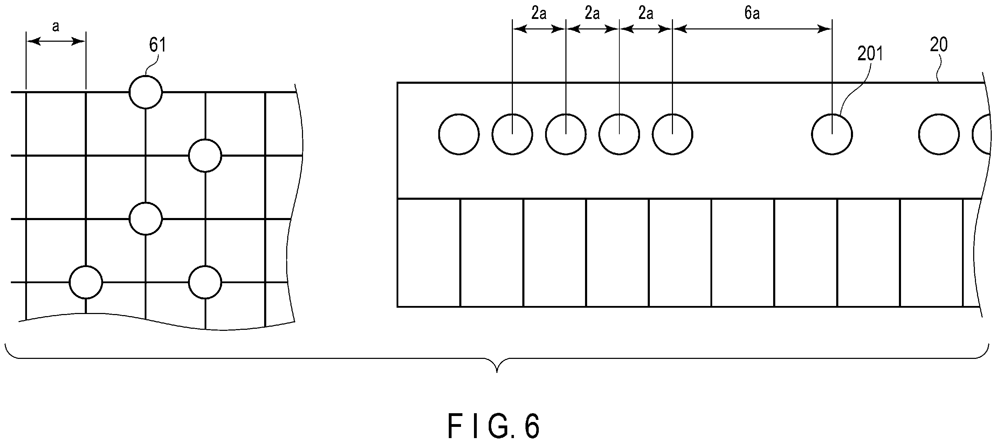

The relationship between the interval between the connection ports 201 and the arrangement of the valves 208 will be described next with reference to FIG. 6.

The left view of FIG. 6 is a conceptual view showing the arrangement interval of the valves arranged in the cartridge 20. The right view of FIG. 6 is a conceptual view showing the interval between the connection ports 201 formed in the cartridge 20. Each valve 208 arranged in the cartridge 20 is arranged at a position corresponding to an intersection of a grid formed by dividing a surface (a yz plane in FIG. 2) on which the flow channel of the cartridge 20 extends into a grid. In the example shown in FIG. 6, a valve position 61 arranged at a position corresponding to an intersection of the grid is indicted by a circle. Letting "a" be the minimum interval of the grid in the moving direction (y-axis direction) of the cartridge 20, the valve positions 61 are arranged at least at the interval "a" concerning the moving direction of the cartridge 20.

On the other hand, the interval between the connection ports 201 of the cartridge 20 is set to an integer multiple of the minimum interval in the arrangement of the valve positions 61. In FIG. 6, the connection ports 201 are arranged at intervals "2a" and "6a". The present invention is not limited to this, and the arrangement interval of the valve positions 61 need only be an integer multiple of a, such as "3a" or "5a". With this, in a case where, for example, the liquid feeding pad 111 of the liquid feeding mechanism 11 does not move, and the cartridge 20 moves to switch connection between the connection port 201 and the liquid feeding pad 111, opening/closing control of the valves by the liquid feeding mechanism 11 can be designed based on "a" as a reference. Hence, since liquid feed control to the cartridge 20 and opening/closing control of the valves 208 can be designed based on a predetermined interval as a reference, a control mechanism associated with liquid feed can be made simple and easy.

Note that depending on the design of the driving mechanism of the conveyance mechanism 12 and the liquid feeding pad 111, the interval may not be an integer multiple of the minimum interval of the arrangement of the valve positions 61. For example, the positions of the connection ports 201 may be set at an interval corresponding to a distance of a fractional precision of the predetermined interval a, for example, "a/2".

An example of the valve positions arranged in the cartridge 20 will be described next with reference to FIG. 7.

FIG. 7 is a side view showing the cartridge 20 viewed from the x-axis direction. FIG. 7 shows the arrangement of the valve positions 61 in a case where a grid as shown in FIG. 6 is assumed. Here, a connecting point 70 indicating a position where the connection port 201 and the liquid feeding pad 111 are connected is indicated by an arrow. If the liquid feeding pad 111 exists at the connecting point 70 indicating the position where the liquid feeding pad 111 is connected to the connection port, the positions of the valves that should be opened/closed to ensure a liquid feeding path are expressed by hatching as opening/closing target valve positions 71.

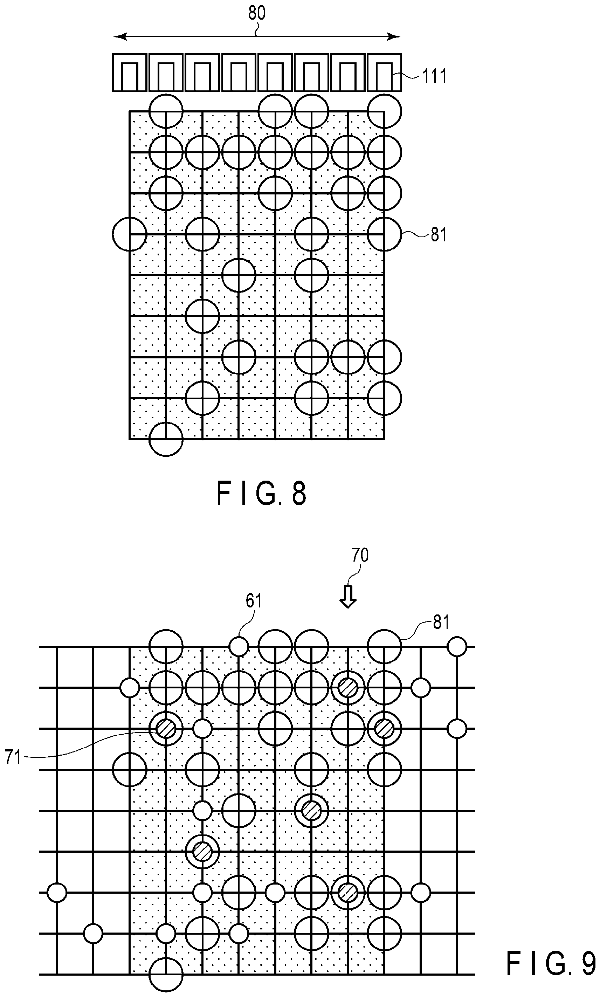

An example of the arrangement of the valve opening/closing mechanisms 13 will be described next with reference to FIG. 8.

FIG. 8 is a view showing the arrangement of the valve opening/closing mechanisms 13. On the grid assumed as in FIG. 7, an opening/closing mechanism position 81 indicating the arrangement of the valve opening/closing mechanism 13 is indicated by a circle at an intersection of the grid. Also, a movable range 80 of the liquid feeding pad 111 is shown on the upper side of the grid.

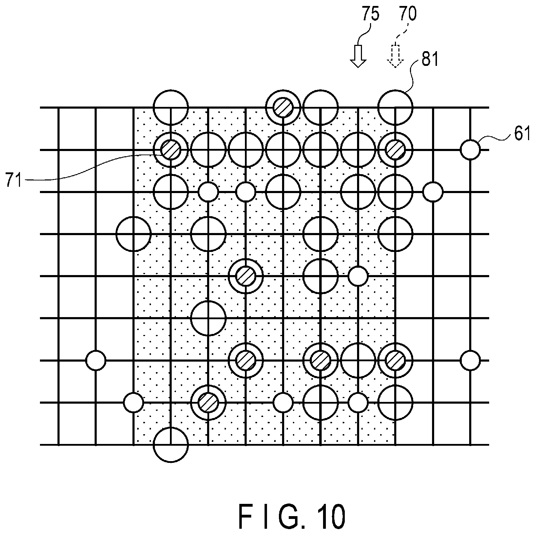

The positional relationship between the valve positions 61 and the opening/closing mechanism positions 81 of the valve opening/closing mechanisms 13 in the cartridge 20 at the time of valve opening/closing control will be described next with reference to FIGS. 9 and 10.

FIG. 9 shows a first positional relationship between the valve positions 61 and the opening/closing mechanism positions 81 at the connecting point 70 of the liquid feeding pad 111. The valve opening/closing mechanisms 13 of the liquid feeding mechanism 11 are arranged in a one-to-one correspondence with the opening/closing target valve positions 71 among the plurality of valve positions 61. Here, valves corresponding to six valve positions 61 are controlled to be opened/closed.

FIG. 10 shows a second positional relationship between the valve positions 61 and the opening/closing mechanism positions 81 at a connecting point 75 of the liquid feeding pad 111. The connecting point 75 indicates a position of the cartridge 20 moved in the rightward direction by the predetermined interval a. In this case, valves corresponding to eight valve positions are controlled to be opened/closed.

Thus, by the connection control function 103, at least one of the liquid feed control unit and the cartridge 20 is moved in accordance with the connecting point where the liquid feeding pad 111 is connected to the connection port 201 such that, among the plurality of valves, one or more opening/closing target valve positions 71 necessary for switching of the flow channel 207 face the opening/closing mechanism positions 81 of the valve opening/closing mechanisms 13. The valve opening/closing mechanisms 13 are arranged to cope with each step of the cell treatment executed in the cartridge 20 even if the connection position changes, and the opening/closing target valve positions 71 change.

As shown in FIGS. 8 to 10, a minimum arrangement capable of covering opening/closing of the flow channel in accordance with movement of the cartridge 20 and the liquid feeding pad 111 is set. For example, by superimposing the opening/closing target valve positions at each connecting point in the cartridge 20 on the grid as a preprocess, the opening/closing mechanism positions 81 of the minimum arrangement can be designed, and the configuration can be simplified.

Note that the valve opening/closing mechanisms 13 may be arranged at all intersections of the grid on the whole surface of the liquid feeding mechanism 11. In this case, the opening/closing mechanism positions 81 need not be initialized in accordance with the cartridge 20.

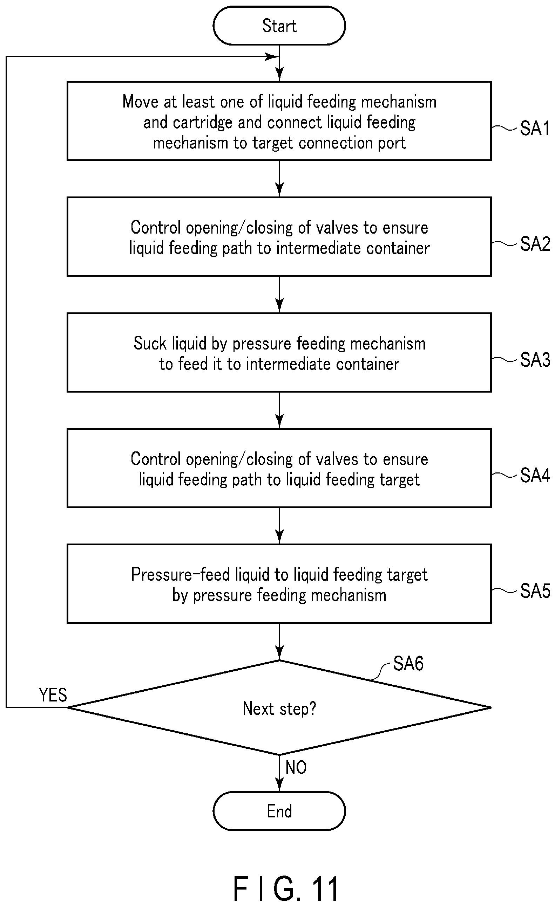

An example of liquid feed control processing of the cell treatment apparatus 3 according to this embodiment will be described next with reference to the flowchart of FIG. 11.

In step SA1, the connection control function 103 moves at least one of the liquid feeding mechanism 11 and the cartridge 20 via the liquid feeding mechanism 11 and the conveyance mechanism 12 to connect the liquid feeding pad 111 to the target connection port 201. Which one of the liquid feeding pad 111 and the cartridge 20 is to be moved is set by the connection control function 103 in advance based on the positional relationship between the valve opening/closing mechanisms and the valves in the cartridge 20. For example, if the process advances to the next step while keeping the positional relationship in the current step between the valve opening/closing mechanisms and the valves in the cartridge 20, and the opening/closing target valves and the valve opening/closing mechanisms are in a one-to-one correspondence in the next step, the liquid feeding pad 111 is moved without moving the cartridge 20. In this way, the moving target (the liquid feeding pad 111 or the cartridge 20) and the position to move (moving amount) are decided in advance based on the positional relationship between the opening/closing target valves and the valve opening/closing mechanisms in each step, and the connection control function 103 executes moving control based on the decided driving target and driving position.

In step SA2, the valve control function 102 controls, via the valve opening/closing mechanisms 13, opening/closing of the valves 208 to ensure the liquid feeding path to the intermediate container 202 in the cartridge 20. That is, opening/closing control of the valves existing at the opening/closing target valve positions 71 shown in FIG. 7 is executed.

In step SA3, the liquid feed control function 101 sucks a liquid via the liquid feeding pad 111 of the liquid feeding mechanism 11 such that the liquid is fed to the intermediate container 202. If the liquid needs to be pressure-fed to the intermediate container 202 by pressure feeding, the liquid feed control function 101 pressure-feeds the liquid via the liquid feeding pad 111.

In step SA4, the valve control function 102 controls, via the valve opening/closing mechanisms 13, opening/closing of the valves 208 to ensure the liquid feeding path to the liquid feeding target in the cartridge 20.

In step SA5, the liquid feed control function 101 pressure-feeds the liquid via the liquid feeding pad 111 of the liquid feeding mechanism 11 such that the liquid is fed to the liquid feeding target.

In step SA6, the connection control function 103 determines whether it is necessary to advance to the next step of the cell treatment. For example, the execution count and the treatment time concerning suction and pressure feed in a case where a suspension containing separated white blood cells is fed to the recovery container or in a case where cells are pressure-fed to the culture container 206 are scheduled in advance, and if the execution count and the treatment time reach the predetermined execution count and treatment time, it is determined to advance to the next step.

Upon determining to advance to the next step, the process advances to step SA1, and the connection control function 103 moves at least one of the liquid feeding mechanism 11 and the cartridge 20 to connect the liquid feeding pad 111 to the connection port 201 associated with the next step. On the other hand, upon determining not to advance to the next step, the cell treatment is ended.

Note that since the processing steps shown in FIG. 11 are merely examples, and an example in which the intermediate container 202 is interposed as a liquid feeding path has been described, an example in which suction processing and pressure feeding processing are performed is shown. However, the present invention is not limited to this, and processing can similarly be performed even if liquid feeding processing including only pressure feeding exists. For example, if the liquid can be fed directly from the liquid feeding target in the preceding step to the liquid feeding target in the current step, opening/closing of the valves is controlled such that the liquid feeding path from the liquid feeding target in the preceding step to the liquid feeding target in the current step is ensured, and the liquid is pressure-fed without performing suction processing.

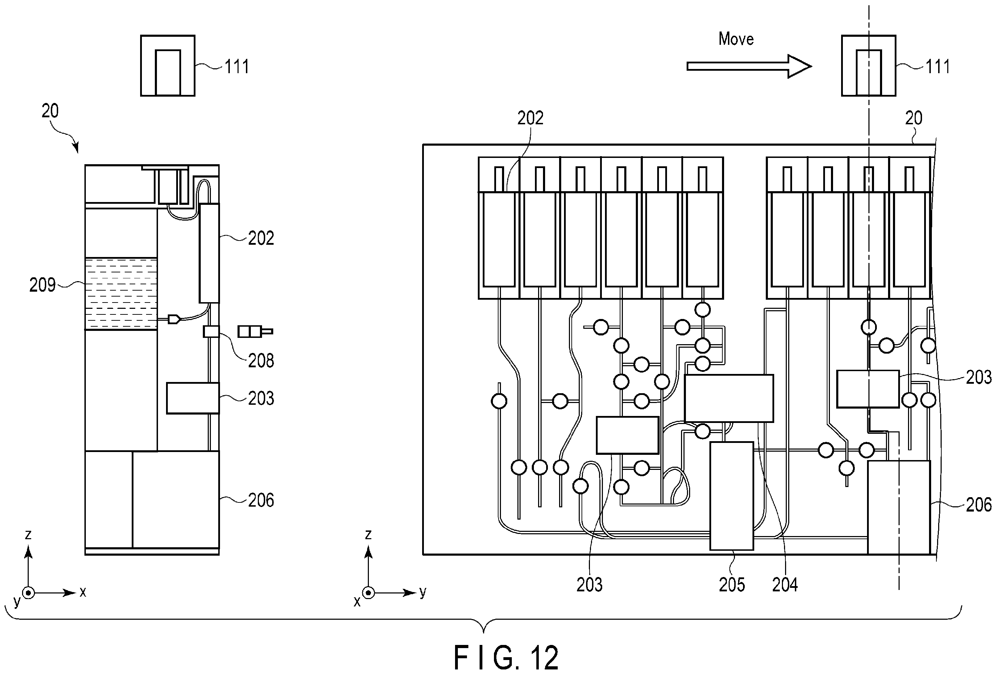

The processing of each of steps SA1 to SA5 in the flowchart of FIG. 11 will be described next with reference to the diagrams of FIGS. 12 to 15.

FIG. 12 corresponds to moving processing of step SA1 in FIG. 11. The diagram of FIG. 12 shows an example in which, to connect the liquid feeding pad 111 to a connection port, at least one of the liquid feeding pad 111 and the cartridge 20 is moved.

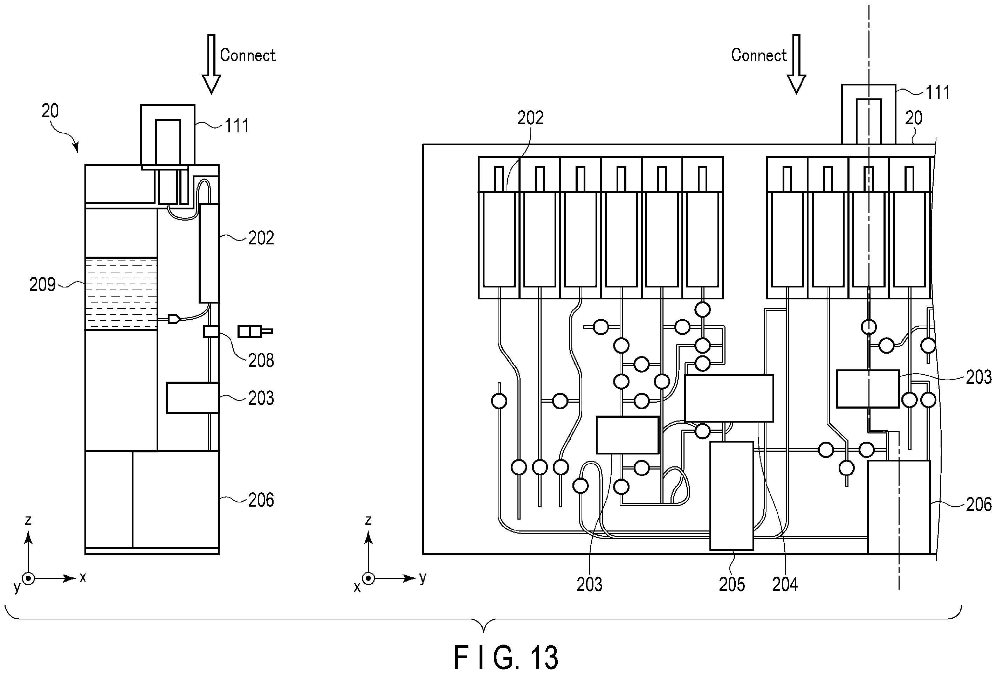

FIG. 13 corresponds to connection processing of step SA1 in FIG. 11, and shows an example in which, to connect the liquid feeding pad 111 to a connection port, at least one of the liquid feeding pad 111 and the cartridge 20 is moved. The connection control function 103 moves the liquid feeding pad 111 or the cartridge 20 to a position where the liquid feeding pad 111 and the connection port 201 can be connected, and the liquid feeding pad 111 then moves vertically in the z-axis direction and is connected to the connection port 201. As for the connection method, the liquid feeding pad 111 may be formed to be fitted on/in the connection port 201, or the liquid feeding pad 111 may be connected by pressing the portion of the connection port 201 with a predetermined pressure. That is, any method can be used if it can maintain airtightness to such an extent that it exerts no influence on pressure feeding and suction from the liquid feeding pad.

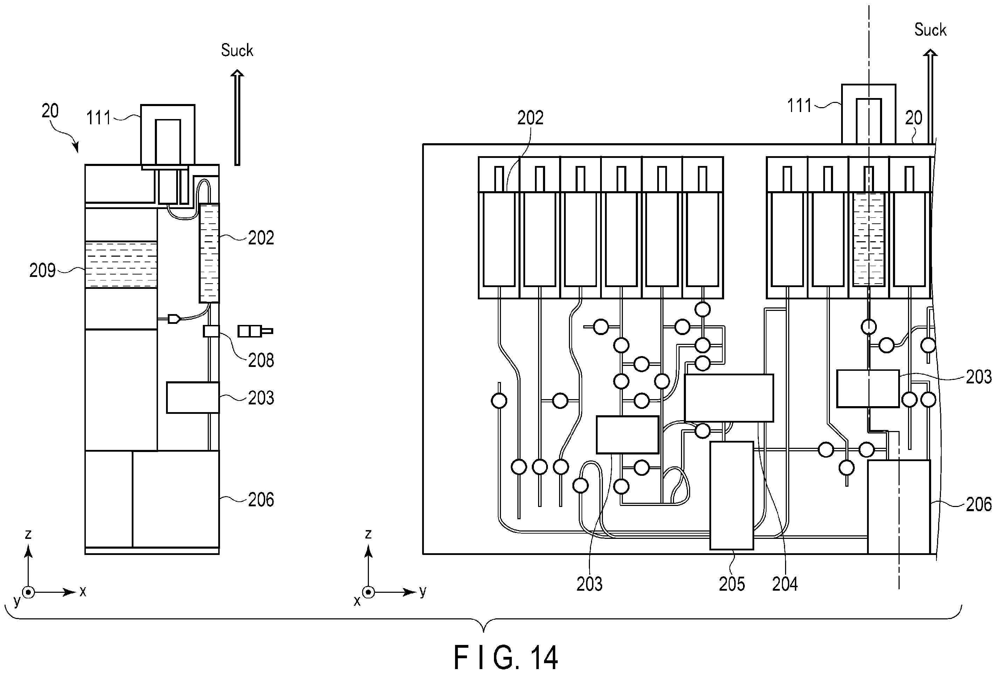

FIG. 14 corresponds to valve opening/closing processing of step SA2 and suction processing of step SA3 in FIG. 11. Here, the valves are controlled to feed the liquid from the liquid pack storage portion 209 to the intermediate container 202. Here, the valve 208 in the left view of FIG. 14 is closed. The liquid feeding pad 111 sucks the liquid by an ejector (convum), thereby feeding the liquid from the liquid pack storage portion 209 to the intermediate container 202.

FIG. 15 corresponds to valve opening/closing processing of step SA4 and pressure feeding processing of step SA5 in FIG. 11. Here, the valves are controlled to feed the liquid from the intermediate container 202 to the culture container 206 that is the liquid feeding target. Here, the valve 208 in the left view of FIG. 15 is opened. If a compressor applies a pressure, air is pushed out from the liquid feeding pad 111, and the liquid is pushed out from the intermediate container 202 and consequently fed to the culture container 206.

An example of the opening/closing mechanism of the valve will be described next with reference to FIGS. 16 and 17.

FIG. 16 shows an example of a magnet valve in which the valve 208 in the cartridge 20 and the valve opening/closing mechanism 13 are each formed using a magnet, and shows a case in which the valve 208 is in a "closed" state.

In the housing of the cartridge 20, a magnet 2082 is attracted to a magnetic member 2081 by a magnetic force, and the flow channel 207 is sandwiched therebetween. The flow channel 207 is crushed because it is made of a flexible material, and the valve 208 is set in a "closed" state. The magnetic member 2081 is assumed to be, for example, an iron component such as a screw made of iron and need only be a magnetic body.

On the other hand, the valve opening/closing mechanism 13 includes a magnet 1121 and a piston portion 1122. If the valve 208 is in the "closed" state, the magnet 1121 retreats to a distance at which the magnet 2082 and the magnet 1121 do not attract each other.

FIG. 17 shows a case in which the valve 208 is in the "open" state. The magnet 1121 is expanded by the piston portion 1122 to a position where the magnet 1121 and the magnet 2082 attract each other, and the magnet 1121 and the magnet 2082 attract each other across the surface portion (housing portion) of the cartridge 20. Since the crushed flow channel 207 returns to a diameter capable of feeding the liquid, the valve 208 is set in the "open" state.

Note that the valve opening/closing mechanism of the valve 208 is not limited to the magnet type. For example, if the housing of the cartridge 20 is made of a flexible material, the valve 208 may be of a lever (handle) type or a push type. More specifically, if the valve 208 is of a lever type, the valve opening/closing mechanism 13 includes a grip mechanism, and the grip mechanism grips and rotates the lever, thereby controlling opening/closing of the valve. If the valve 208 is of a push type, the valve opening/closing mechanism 13 causes the piston portion 1122 to push in the valve 208, thereby controlling opening/closing of the valve.

Note that pipes of one end of a manifold may be connected to the connection ports 201 of the cartridge 20, respectively, and the liquid feeding pad 111 may be connected to the other end where the pipes are integrated. This makes it possible to implement liquid feed control only by moving the liquid feeding pad 111 on the manifold without moving the cartridge 20. Furthermore, the longer the length of the manifold in the longitudinal direction, the narrower the moving range of the liquid feeding pad 111 can be. This can improve the efficiency of driving control

According to the above-described embodiment, using the conveyor on which the cartridge including the plurality of connection ports is mounted, and the liquid feed control unit connected to at least one of the plurality of connection ports and configured to control liquid of the liquid in the cartridge, when connecting the liquid feed control unit and the cartridge, the cell treatment apparatus moves at least one of the liquid feed control unit and the cartridge. Since this makes it possible to execute liquid feed for all the connection ports of the cartridge using one liquid feeding pad, the cost of the apparatus can be reduced without preparing the same number of liquid feeding pads as the number of the plurality of connection ports.

In addition, the valve opening/closing mechanisms and the valves in the cartridge are configured in a one-to-one correspondence to implement switching of the liquid feeding path in each step of liquid feed, and are arrayed in the longitudinal direction at an interval corresponding to an integer multiple of a predetermined interval. Hence, even if at least one of the liquid feed control unit and the cartridge moves, and the connecting point between the liquid feeding pad and the connection port of the cartridge changes, desired switching of the liquid feeding path can be implemented.

Note that the term "processor" used in the above explanation means, for example, a Central Processing Unit (CPU), a Graphics Processing Unit (GPU), or a circuit such as an Application Specific Integrated Circuit (ASIC) or a programmable logic device (for example, a Simple Programmable Logic Device (SPLD), a Complex Programmable Logic Device (CPLD), or a Field Programmable Gate Array (FPGA)). If the processor is, for example, a CPU, the processor implements a function by reading out a program stored in a storage circuit and executing it. On the other hand, if the processor is an ASIC, the function is directly incorporated as a logic circuit in the circuit of the processor, instead of storing the program in the storage circuit. Note that the processor according to this embodiment is not necessarily configured as a single circuit for each processor, and a plurality of independent circuits may be combined to form one processor and implement the function. Furthermore, a plurality of constituent elements in the drawings may be integrated to form one processor and implement the function. That is, it is possible to execute efficient and accurate inspection.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A cell treatment apparatus (3) comprising:
a conveyance mechanism (12) capable of moving a cartridge (20) including a plurality of connection ports (201) ;
a liquid feed control unit (11, 101) connected to at least one of the plurality of connection ports (201) and configured to control feed of a liquid in the cartridge (20); and
a connection control unit (103) configured to move at least one of the liquid feed control unit (11, 101) and the cartridge (20) when connecting the liquid feed control unit (11, 101) and the cartridge (20).

2. The cell treatment apparatus (3) according to claim 1, further comprising one or more valve opening/closing units (13) each configured to open/close a plurality of valves arranged in the cartridge (20) to switch a flow channel of the liquid arranged in the cartridge (20).

3. The cell treatment apparatus (3) according to claim 2, wherein the liquid feed control unit (11, 101) includes a liquid feeding pad (111) that is a portion connected to one of the connection ports (201) and is movable, and
the connection control unit (103) moves at least one of the liquid feed control unit (11, 101) and the cartridge (20) such that one or more target valves necessary for switching of the flow channel among the plurality of valves face the valve opening/closing units (13) in accordance with a connecting point where the liquid feeding pad (111) is connected to one of the connection ports (201).

4. The cell treatment apparatus (3) according to claim 2 or 3, wherein the valve opening/closing units (13) are immovably arranged.

5. The cell treatment apparatus (3) according to any one of claims 2 to 4, wherein a minimum interval between the connection ports (201) is an integer multiple of a minimum interval between the valves.

6. The cell treatment apparatus (3) according to any one of claims 3 to 5, wherein a maximum moving distance of the liquid feeding pad (111) is shorter than a maximum array length of the plurality of connection ports (201) arranged in the cartridge (20).

7. The cell treatment apparatus (3) according to any one of claims 1 to 6, wherein the liquid feed control unit (11, 101) executes the liquid feed by suction and pressure feed.

8. The cell treatment apparatus (3) according to any one of claims 2 to 7, wherein the valve opening/closing units (13) are two-dimensionally arrayed in a matrix.

9. The cell treatment apparatus (3) according to any one of claims 2 to 8, wherein the number of liquid feeding pads (111) is smaller than the number of the plurality of connection ports (201).

10. A cell treatment method using a cell treatment apparatus including a conveyance mechanism capable of moving a cartridge including a plurality of connection ports, and a liquid feed control unit connected to at least one of the plurality of connection ports and configured to control feed of a liquid in the cartridge, the method comprising
moving at least one of the liquid feed control unit and the cartridge when connecting the liquid feed control unit and the cartridge.

11. The cell treatment method according to claim 10, further comprising opening and closing a plurality of valves arranged in the cartridge by using one or more valve opening/closing units to switch a flow channel of the liquid arranged in the cartridge.

12. The cell treatment method according to claim 11, wherein at least one of the liquid feed control unit and the cartridge moves such that one or more target valves necessary for switching of the flow channel among the plurality of valves face the valve opening/closing units in accordance with a connecting point where a liquid feeding pad is connected to one of the connection ports, the liquid feeding pad being a portion connected to one of the connection ports and being movable.

13. The cell treatment method according to claim 11 or 12, wherein the valve opening/closing units are immovably arranged.

14. The cell treatment method according to any one of claims 11 to 13, wherein a minimum interval between the connection ports is an integer multiple of a minimum interval between the valves.

15. The cell treatment method according to any one of claims 11 to 14, wherein a maximum moving distance of the liquid feeding pad is shorter than a maximum array length of the plurality of connection ports arranged in the cartridge.
